(19) Europäisches Patentamt
European Patent Office
Office européen des brevets

(11) Veröffentlichungsnummer: **0 577 979 B1**

(12) # EUROPÄISCHE PATENTSCHRIFT

(45) Veröffentlichungstag der Patentschrift: **18.10.95**

(51) Int. Cl.[6]: **C07C 43/192**, C07C 43/17, C08F 16/32

(21) Anmeldenummer: **93108998.1**

(22) Anmeldetag: **04.06.93**

(54) **Cyclopolymerisierbare Fluorverbindungen, Verfahren zu deren Herstellung und daraus erhältliche thermoplastische Fluorpolymere.**

(30) Priorität: **17.06.92 DE 4219764**

(43) Veröffentlichungstag der Anmeldung:
**12.01.94 Patentblatt 94/02**

(45) Bekanntmachung des Hinweises auf die Patenterteilung:
**18.10.95 Patentblatt 95/42**

(84) Benannte Vertragsstaaten:
**BE DE FR GB IT**

(56) Entgegenhaltungen:
**EP-A- 0 303 298**
**EP-A- 0 303 298**
**US-A- 2 917 548**
**US-A- 3 250 808**
**US-A- 3 655 765**

(73) Patentinhaber: **BAYER AG**

**D-51368 Leverkusen (DE)**

(72) Erfinder: **Krüger, Ralf, Dr.**
**Wolfskaul 3**
**D-51061 Köln (DE)**
Erfinder: **Negele, Michael, Dr.**
**Ratinger Weg 8**
**W-5650 Solingen 11 (DE)**
Erfinder: **Marhold, Albrecht, Dr.**
**Carl-Duisberg-Strasse 329**
**W-5090 Leverkusen 1 (DE)**

EP 0 577 979 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft 2-Allyl-perfluor-(cyclo)alkyltrifluorvinylether, ein Verfahren zu deren Herstellung und daraus hergestellte Homo- und Copolymerisate.

Die erfindungsgemäßen Monomeren sind durch radikalische Polymerisation unter Ausbildung von Ringstrukturen in der Polymerkette polymerisierbar (Cyclopolymerisation).

Die Polymerisate ergeben amorphe, transparent Beschichtungen oder Formkörper mit Glasübergangstemperaturen zwischen 60 und 150 °C und besitzen ein gutes Löslichkeitsverhalten in einer Reihe von organischen Lösungsmitteln. Letzteres erweist sich besonders dadurch als vorteilhaft, daß ein Auftrag auf eine zu beschichtende Oberfläche ebenso leicht möglich ist wie die spätere Ablösung, so daß die Recyclingfähigkeit der mit den erfindungsgemäßen Fluorpolymeren beschichteten Werkstoffe erhöht wird.

Bei der Beschichtung von anderen polymeren Werkstoffen, z.B. in Form von Lichtleiterfasern, mit Fluorpolymeren tritt oft das Problem auf, daß wegen nicht vorhandener Löslichkeit und hoher Schmelz- oder Erweichungstemperatur des Fluorpolymeren der Auftrag bei einer für den betreffenden Werkstoff zu hohen Temperatur erfolgen muß, so daß in der Regel die Thermostabilität und/oder Wärmeformbeständigkeit bei der hohen Belastung nicht mehr gegeben sind.

Amorphe, hochtransparente Fluorpolymere mit cyclischen Ketten-Strukturelementen sind z.B. aus US-A 4 975 505 bekannt. Jedoch weisen diese Polymerisate für viele Anwendungen zu hohe Glasübergangstemperaturen von mindestens 150 °C auf und zeigen zudem nur in ausgewählten fluorierten Verbindungen ein befriedigendes Löslichkeitsverhalten.

Die EP-A 303 292 beschreibt durch Cyclopolymerisation erhältliche amorphe Fluorpolymere, die lediglich Glasübergangstemperaturen von unter 100 °C aufweisen und ebenfalls nicht in gängigen organischen Solventien löslich sind.

Gegenstand der vorliegenden Erfindung sind 2-Allyl-perfluoralkyltrifluorvinylether der nachfolgenden Formel (I)

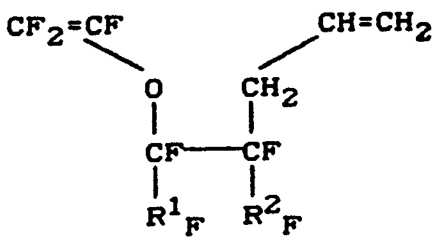

(I)

wobei

$R^1_F$ und $R^2_F$ entweder $-CF_3$ oder gemeinsam $(CF_2)_n$ mit n = 2 oder 3 bezeichnen.

Bevorzugt sind Monomere der Formel (I) mit n = 3.

Bei der radikalischen Polymerisation werden Kettenstrukturen mit folgenden Bausteinen erzeugt:

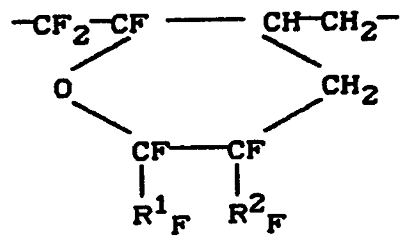

(II)

oder

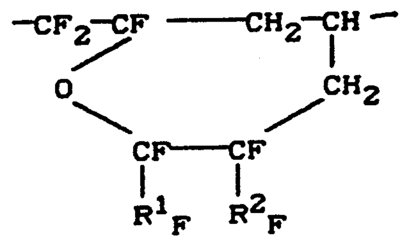

(III)

oder

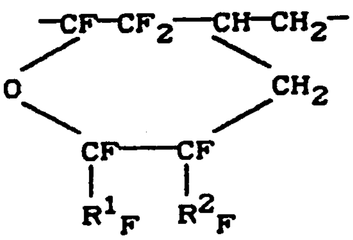

(IV)

In Abhängigkeit von der Polymerisationstemperatur werden Polymerisationsgrade erreicht, die einem Restgehalt an C-C-Doppelbindungen von unter 10 % bis 2 % der Anzahl der Doppelbindungen des Monomeren entsprechen.

Das erfindungsgemäße Verfahren zur Herstellung der erfindungsgemäßen Monomere ist dadurch gekennzeichnet, daß

a) entsprechende 2-Allyl-substituierte Perfluorcycloketone bzw. Perfluorketone mit Hexafluorpropenoxid umgesetzt werden,

b) die erhaltenen 2-(Allylperfluorcycloalkoxy)- bzw. 2-)Allylperfluoralkoxy)-perfluorpropancarbonsäurefluoride durch alkalische Verseifung in deren Natriumsalze überführt werden und

c) die Natriumsalze bei 150 - 350 °C decarboxyliert werden.

Vorzugsweise werden zur Reduzierung der Entstehung von 2-Allyl-perfluoralkyl-1,1,1,2-Tetrafluorethylether als Nebenprodukt die unter b) erhaltenen Natriumsalze in aromatischen Kohlenwasserstoffen gelöst, unlösliche Salze aus der Lösung abgetrennt und die Natriumsalze azeotrop getrocknet.

Die zur Herstellung der erfindungsgemäßen Monomeren benötigten 2-Alkyl-substituierten Perfluorcycloketone bzw. Perfluorketone sind an sich bekannt; siehe z.B. Tetrahedron 23, 4243 (1967); US-A 3 655 765.

Es war aber durchaus überraschend, daß die Umsetzung dieser Ausgangsverbindungen mit Hexafluorpropenoxid trotz der sterischen Hinderung der Carbonyl-Gruppe durch den 2-Allyl-Substituenten mit befriedigenden Ausbeuten möglich ist.

Erfindungsgemäß wird die Umsetzung in einem geeigneten Lösungsmittel, das ein salzbildendes Mittel enthält, unterhalb Raumtemperatur durchgeführt, anschließend das Lösungsmittel abdestilliert und das Grobdestillat fraktioniert.

Für die Überführung der 2-Allyl-substituierten Perfluorcycloalkoxy- bzw. Perfluoralkoxy-perfluorpropancarbonsäurefluoride in die erfindungsgemäßen 2-Allyl-substituierten Perfluorcycloalkyltrifluorvinyl- bzw. Perfluoralkyltrifluorvinylether werden die Carbonsäurefluoride durch alkalische Verseifung in die Natriumsalze überführt und diese Natriumsalze bei 150 bis 350 °C, bevorzugt bei 180 bis 240 °C decarboxyliert.

Als Nebenprodukt entstehen erhebliche Mengen an 1,1,1,2-Tetrafluorethyl-Verbindungen, die schlecht oder nur mit erheblichem Aufwand abzutrennen sind.

Für die erfindungsgemäßen Verbindungen wurde ein Verfahren gefunden, die primär hergestellten Natriumsalze in aromatischen Kohlenwasserstoffen, bevorzugt Toluol, zu lösen, unlösliche Salze abzutrennen und die Natriumsalze durch Abdestillieren azeotrop zu trocknen. Dadurch werden Wasser und alkalische Verunreinigungen, die das Auftreten der unerwünschten 1,1,1,2-Tetrafluorethyl-Verbindungen begünstigen, weitgehend abgetrennt.

Es gelingt so, die erfindungsgemäßen 2-Allyl-substituierten Perfluorcycloalkyltrifluorvinyl- bzw. Perfluoralkyltrifluorvinylether in hoher, für Polymerisationsversuche ausreichender Reinheit herzustellen.

Die Herstellung der erfindungsgemäßen Polymerisate erfolgt auf radikalischem Wege. Ansonsten gibt es keinerlei Beschränkung hinsichtlich der Polymerisationsmethode. Es kann in Substanz, Lösung (geeignete Lösungsmittel sind Fluorkohlen(wasser)stoffe, z.B. Hexafluorcyclopentan, Perfluorbutan oder Fluorchlorkohlenstoffe, z.B. Trichlortrifluorethan), Suspension oder Emulsion polymerisiert werden. Der Radikalstart kann durch energiereiche Strahlung, thermisch oder durh Radikalinitiatoren eingeleitet werden. Zur chemischen Initiierung werden grundsätzlich bekannte und für das jeweilige Reaktionsmedium geeignete Verbindungen eingesetzt.

So verwendet man bei der Substanz-Lösungs- und Suspensionspolymerisation organische, öllösliche Peroxide, die auch fluoriert sein können, wie Benzoylperoxid, Trifluoracetylperoxid oder organische lösliche Azoverbindungen, wie Azobisisobutyronitril. Bei der Emulsionspolymerisation, die für die Herstellung der erfindungsgemäßen Copolymere bevorzugt wird, werden wasserlösliche anorganische Perverbindungen als Initiatoren benutzt, wie Persulfate, Perborate, Percarbonate etc., im allgemeinen in Form ihrer Natrium- oder Ammoniumsalze.

In Abhängigkeit von der Polymerisationstemperatur und von der Zerfallskonstante des Initiators müssen bei Anwendung niederer Temperaturen zur Polymerisation Zerfallsbeschleuniger, in der Regel Reduktionsmittel, zusätzlich eingesetzt werden. Als solche können dienen: Schwefelverbindungen, wie etwa Natriumsulfit, Natriumpyrosulfit oder Rongalit C (Natriumformamidinsulfinsäure), weiterhin organische Reduktionsmittel, wie Ascorbinsäure, Metallsalze, wie Eisen (II)- oder Kobalt-(II)-salze, metallorganische Verbindungen etc.

Die Reaktionstemperaturen für die Copolymerisation liegen zwischen -15 und +120 °C, bevorzugt bei 0 bis 90 °C.

Für die Copolymerisation der erfindungsgemäßen Monomeren (I) kommen folgende Comonomere (II) in Frage:

Ethylene mit mindestens einem Fluoratom (Vinylfluorid, Vinylidenfluorid, Trifluorethylen, Tetrafluorethylen, Trifluorchlorethylen)

Monomere vom Typ $CF_2 = CYX$, wobei Y = H, F, oder Cl und X einen aliphatischen perfluorierten Rest bedeuten kann (z.B. Hexafluorpropen, Hydro- oder Chlorpentafluorpropen) oder ein $R_F$-O-Rest sein kann (z.B. Perfluormethyl-perfluorvinylether; Perfluorcyclopentyl-perfluorvinylether).

Die Copolymerisate können je nach gewünschten Eigenschaften 1 bis 99 mol-% des erfindungsgemäßen Monomeren (I) enthalten. Die Menge an Monomer (I) sollte mindestens 20 mol-% betragen, wenn es mit Comonomeren, die kristallisationsfähige Polymere bilden können, zu einem amorphen Polymer copolymerisiert werden soll.

Die Copolymerisation mit gasförmigen Monomeren wird unter einem erhöhten Druck durchgeführt. Dieser Druck soll mindestens 2 bar betragen, braucht aber den Wert von 100 bar nicht zu überschreiten.

## Beispiele

### Beispiel 1

a) (2-Allyl-)heptafluorcyclopentanon

Es werden bei 0 bis 5 °C (Eisbad) 750 g (3,54 mol) Octafluorcyclopenten vorgelegt, 205 g (3.59 mol) KOH-Pulver zugesetzt und diese Suspension aufgerührt. Dann werden innerhalb von 3 h 211 g (3,64 mol) Allylalkohol zugetropft, die leichte Exothermie kann mit dem Eisbad abgefangen werden. 16 h bei Raumtemperatur nachrühren, dann in 1.500 ml Wasser einrühren, organische Phase abtrennen und über $Na_2SO_4$ trocknen.

Rohausbeute 785 g (3,14 mol) = 88,7 % d. Th.

Das Rohmaterial wird an einer Normaldruckdestillation an einer 30 cm Vigreux-Kolonne mit Kolonnenkopf unterzogen. Nach Abnahme von Leichtsiedern (Octafluorcyclopenten, ca. 32 g = 0,151 mol) wird 4 h bei Totalrücklauf am Rückfluß gekocht, bis die Sumpftemperatur auf ca. 140 °C angestiegen ist. (Umlagerung des primär gebildeten 1-Allyloxyheptafluorcyclopenten in (2-Allyl-)heptafluorcyclopentanon;

vergl. Tetrahedron, 23, 4243 (1967); US-Pat. 3 655 765).

Jetzt erfolgt die eigentliche Produktdestillation

| | |
|---|---|
| Vorlauf: | Kp $_{ND}$ bis 110 °C 67 g (GC 63 % Produkt, Rest Octafluorcyclopenten und wenig Allylether) |
| Hauptfraktion: | Kp $_{ND}$ 112 °C 615 g (2,46 mol = 69,4 %) |
| Sumpf: | 53 g |

b) 2-[2-(Allyl-)octafluorcyclopentoxy]perfluorpropancarbonsäurefluorid

In 500 ml Diglyme werden 150 g (2,59 mol) geglühtes Kaliumfluorid suspendiert und unter Rühren bei 5 bis 20 °C innerhalb von 90 min 504 g (2,0 mol) (2-Allyl-)heptafluorcyclopentanon zugetropft. Nach Abklingen der leicht exothermen Reaktion wird auf 5 °C gekühlt und Hexafluorpropenoxid so rasch einkondensiert, daß an einem Trockeneiskühler nur schwacher Rückfluß auftritt. Nachdem 330 g (ca. 1,99 mol) Hexafluorpropenoxid eingeleitet werden (ca. 80 g pro h) wird über Nacht bei Raumtemperatur nachgerührt.

Anschließend werden Produkt und Anteile Diglyme im Wasserstrahlvakuum bis $Kp_{18}$ ca. 50 °C abdestilliert. Roh 940 g.

Das Grobdestillat wird an einer 30 cm Füllkörper-Kolonne fraktioniert:

Nach einem Vorlauf (102 g) von nicht umgesetztem Einsatzmaterial werden als Hauptfraktion ($Kp_{15}$ 41 bis 44 °C) 554 g erhalten, die lt. GC ca. 65 % des Zielproduktes und ca. 30 % Diglyme enthalten.

Ausbeute: 57,8 % (bezogen auf GC-Reinheit)

Im Sumpf verbleiben ca. 15 % des Zielproduktes, Rest Diglyme und Mehrfach-Addukte von HFPO.

$^1$H-NMR: $\delta$ = 2.83 ppm (dm,2H,-$CH_2$-); 5.34 ppm (dm, 2H, =$CH_2$) und 5,8 ppm (m,1H,CH =)-(gg.int.TMS in $CDCl_3$).

c) 2-(Allyl-)octafluorcyclopentyltrifluorvinylether

In 300 ml Dioxan werden 554 g Destillat aus Beispiel 2 [= ca. 360 g (1,02 mol) 2-[2(Allyl)-octafluorcyclopentoxy]perfluorpropancarbonsäurefluorid] bei 0 bis 5 °C vorgelegt, innerhalb von 3 h mit 220 g 45 %iger Natronlauge gegen Phenolphthalein alkalisch gestellt. Es bildet sich eine Suspension, die im Wasserstrahlvakuum auf ca. 1/3 ihres Volumens eingeengt wird. Das Material wird mit 500 ml Toluol verrührt, unlösliche Salze werden abgesaugt und mit Toluol nachgewaschen.

Die Toluollösung wird am Rotationsverdampfer eingeengt, es verbleiben 553 g, die im Ölpumpenvakuum bei max. 50 °C weiter eingeengt werden.

Die verbleibenden 489 g des hochviskosen Natriumsalzes werden im Ölpumpenvakuum auf einem Metallbad langsam auf max. 280 °C erhitzt. Das bis 100 °C Badtemperatur über eine Brücke abdestillierende Material besteht zum größten Teil aus Resten Toluol und Diglyme und wird verworfen. Ab 150 bis 180 °C setzt die thermische Zersetzung des Natriumsalzes ein. Es werden 352 g Destillat aufgefangen, die an einer 30 cm-Füllkörper-Kolonne im Wasserstrahlvakuum fraktioniert werden:

| | |
|---|---|
| Vorlauf: | $Kp_{18}$ < 35 °C; 45 g; verworfen. |
| Fraktion I $Kp_{18}$ | 41 - 48 °C, 189 g, lt. GC 77 % Zielprodukt, 15 % Diglyme |
| Fraktion II $Kp_{18}$ > | 50 °C 57 g größtenteils Diglyme |
| Sumpf: | ca. 50 g, verkreuzt. |

Die Produktfraktion I wird an der gleichen Kolonne redestilliert:

| | |
|---|---|
| Fraktion I $Kp_{18}$ | 45 - 47 °C 155 g, lt. GC 88 % Zielprodukt |
| Sumpf: | ca. 30 g, < 3 % Diglyme |
| $^1$H-NMR: | $\delta$ = 2.79 ppm (dm,2H,-$CH_2$-); 5.30 ppm (dm, 2H, =$CH_2$) und 5.72 ppm (m,1H,CH =)-(gg.int.TMS in $CDCl_3$). |

Die als Verunreinigung auftretende 1,1,1,2-Tetrafluor ethoxy-Verbindung zeigt eine zusätzliche $^1$H-Resonanz bei 6.05 ppm.

Beispiel 2

a) (2-Allyl-)pentafluorcyclobutanon

In 116 g (2 mol) Allylalkohol werden bei -5 °C 320 g (1.98 mol) Hexafluorcyclobuten einkondensiert. Dann werden langsam innerhalb von 3 h 113 g (1.98 mol) KOH, gelöst in 150 ml Wasser zugetropft, wobei die Temperatur auf 0 bis 5 °C gehalten wird. Anschließend wird über Nacht bei Raumtemperatur nachgerührt.

In 1.000 ml Wasser einrühren, organische Phase abtrennen und über $Na_2SO_4$ trocknen.

Rohausbeute: 372 g

Das Rohmaterial wird wie in Beispiel 1 beschrieben thermisch umgelagert, anschließend wird bei Normaldruck fraktioniert.

Kp $_{ND}$: 63-65 °C 289 g (lt. GC 95 %ig; 1.44 mol = 73 %)
Sumpf: 75 g

b) 2-[2-(Allyl-)hexafluorcyclobutoxy]-perfluorpropancarbonsäurefluorid

1 mol des Ketons aus Beispiel 4 wird analog Beispiel 2 mit Hexafluorpropenoxid umgesetzt.

Es wird nach Aufarbeitung eine Produktfraktion ($Kp_{18}$ 34-36 °C) erhalten, die ca. 85 % (GC) an der Zielverbindung ist; die Ausbeute beträgt ca. 60 %.

c) 2-(Allyl)-hexafluorcyclobutyltrifluorvinylether

Das Carbonsäurefluorid (0,5 mol, 85 %ig) aus Beispiel 5 wird analog Beispiel 3 ins Natriumsalz überführt und bei 150-220 °C im Ölpumpenvakuum pyrolysiert. Das Kondensat wird im Wasserstrahlvakuum fraktioniert.

Ausbeute: 27 % bezogen aus eingesetztem Carbonsäurefluorid.

$Kp_{20}$: 32-33 °C lt. GC ca. 82 % am Zielprodukt.

Beispiel 3

a) (3-Allyl-)heptafluorbutan-2-on

In 116 g (2 mol) Allylalkohol werden bei -5 °C 396 g (1,98 mal) Octafluorbut-2-en einkondensiert. Anschließend werden langsam innerhalb von 3 h 113 g (1,98 mol) kOH, gelöst in 150 ml Wasser zugetropft, wobei die Temperatur auf 0-5 °C gehalten wird. Über Nacht bei Raumtemperatur rühren und analog Beispiel 4 aufarbeiten.

Rohausbeute 438 g.

Das Rohmaterial wird wie analog Beispiel 1 thermisch umgelagert und anschließend bei Normaldruck fraktioniert.

Kp $_{ND}$: 87-89 °C 348 g (lt. GC 97 %ig; 1,46 mol = 73,8 %)
Sumpf: 85 g

b) 2-[3-(Allyl-)heptafluorbut-2-oxy]perfluorpropancarbonsäurefluorid

1 mol des Ketons aus Beispiel 7 wird analog Beispiel 2 mit Hexafluorpropenoxid umgesetzt.

Es wird nach Aufarbeitung eine Produktfraktion ($Kp_{18}$ 56-58 °C) erhalten, die ca. 78 % (GC) an der Zielverbindung ist; die Ausbeute beträgt ca. 42 %.

c) 3-(Allyl-)heptafluorbut-2-yl-trifluorvinylether

Das Carbonsäurefluorid (0,5 mol, 78 %ig) aus Beispiel 8 wird analog Beispiel 3 in Natriumsalz überführt und bei 150-230 °C im Ölpumpenvakuum pyrolysiert. Das Kondensat wird im Wasserstrahlvakuum fraktioniert.

Ausbeute: 29 % bezogen auf eingesetztes Carbonsäurefluorid.

Beispiel 4

In einen 100 ml Glaskolben wurden unter Rühren 30 g 1,1,2,2,3,3-Hexafluorcyclopentan, 50 mg Diisopropylperoxydicarbonat und 15 g 2-(Allyl-)octafluorcyclopentyltrifluorvinylether (mit der in Beispiel 1 beschriebenen Reinheit) gegeben und auf -50 °C gekühlt. Die Reaktionsapparatur wurde dann 3 mal bis auf etwa 4 mbar evakuiert und jeweils mit Stickstoff belüftet. Das Reaktionsgemisch wurde unter konstanter Rührung auf 40 °C erwärmt. Nach einer Gesamtreaktionszeit von 40 h bei 40 °C wurde das Gemisch abgekühlt. Es wurde eine viskose farblose Lösung mit einem Feststoffgehalt von 31,4 Gew.-% erhalten und durch Einrühren in Ethanol gefällt. Es wurden 13 g eines weißen pulverförmigen Polymeren isoliert. Das Polymer ist löslich in Aceton. Der durch $^{19}$-F-NMR-Spektroskopie bestimmte Restgehalt an nicht umgesetzten Fluorvinylgruppen beträgt weniger als 9 mol-%, bezogen auf die eingesetztem Fluorvinylgruppen (Auswertung des Signals bei -115 ppm in Aceton-$d_6$ gegen den Standard $CFCl_3$). Im $^1$H-NMR-Spektrum wurde der Gehalt an nicht umgesetzten Allylgruppen zu weniger als 4 mol-%, bezogen auf die eingesetzten Allylgruppen, bestimmt (Auswertung der Signale bei 5,3-5,5 ppm: 2 Dubletts und bei 5,9 ppm: 1 Sextett, gemessen in Aceton-$d_6$).

$[\eta]$ = 0,031 dl/g (Aceton/25 °C)
DSC: $T_g$ = 100 °C

Beispiel 5

In einen 100 ml Glaskolben wurden unter Rühren 40 g entionisiertes Wasser, 0,16 g Lithiumperfluoroctylsulfonat, 0,3 g Kaliumperoxidisulfat und 10 g 2-Allyl-octafluorcyclopentyl-trifluorvinylether (mit der in Beispiel 1 beschriebenen Reinheit) gegeben und auf 1 °C gekühlt. Die Reaktionsapparatur wurde dann 5

mal bis auf etwa 20 mbar evakuiert und jeweils mit Stickstoff belüftet. Das Reaktionsgemisch wurde unter konstanter Rührung auf 70°C erhitzt. Nach einer Gesamtreaktionszeit von 10 h bei 70°C wurde das Gemisch abgekühlt. Nach mehrstündigem Stehen setzten sich am Boden des Reaktionsgefäßes etwa 1 g einer viskosen öligen Phase ab, die abgetrennt wurde. Aus der darüberstehenden milchig-weißen Emulsion wurden durch Koagulation mit einer 4 %igen wäßrigen Magnesiumsulfatlösung 4.5 g eines weißen pulverförmigen Polymeren isoliert. Das Polymer ist in Bistrifluormethylphenol (BTFMP) und Aceton gelfrei löslich. Die Grenzviskositätszahl beträgt 0,042 dl/g (THF/25°C); DSC: Tg = 64°C.

Beispiel 6

Beispiel 5 wird bei 90°C wiederholt. Nach einer Gesamtreaktionszeit von 4 h setzten sich nach Abkühlung und mehrstündigem Stehen am Boden des Reaktionsgefäßes etwa 2 g einer viskosen öligen Phase ab, die abgetrennt wurde. Aus der darüberstehenden milchig-weißen Emulsion wurden durch Koagulation mit einer 4 %igen wäßrigen Magnesiumsulfatlösung 3,5 g eines weißen pulverförmigen Polymeren isoliert. Das Polymer ist in Bistrifluormethylphenol (BTFMP) und Aceton gelfrei löslich. Die Grenzviskositätszahl (Staudingerindex) beträgt 3,5 ml/g (THF/25°C). Das gelchromatographisch in THF bestimmte Gewichtsmittel des Molekulargewichts beträgt $1,3 \cdot 10^4$ g/mol mit einer Uneinheitlichkeit von 0,6 (Polystyrol-Eichkurve).
DSC: $T_g$ = 58°C

Beispiele 7 - 9

Copolymerisation von 2-Allyl-octafluorcyclopentyltrifluorvinylethermit Trifluorchlorethylen

In einem 0,3 l-Autoklaven wurden 130 ml entionisie$_{rtes}$ Wasser vorgelegt. Darin wurden 0,5 g Lithiumperfluoroctylsulfonat gelöst. Mit Lithiumhydroxid wurde diese Lösung auf einen pH-wert von etwa 10 eingestellt. Dann wurde der geschlossene Autoklav 3 mal jeweils mit einem Stickstoffdruck von 10 bar beaufschlagt und anschließend auf Normaldruck entspannt. In den Autoklaven wurden die in Tabelle 1 angegebenen Mengen (= Monomer 1) an 1-2-Allyl-octafluorcyclopentyl-trifluorvinylether (gemäß Beispiel 1) und 30 g Trifluorchlorethylen gegeben und das Reaktionsgemisch unter Rühren auf 70°C erwärmt. Nach Erreichen dieser Temperatur wurden 20 g einer wäßrigen Lösung, die 0,5 g Kaliumperoxidisulfat enthält, in den Autoklaven gedrückt. Nach den in Tabelle 1 angegebenen Gesamtreaktionszeiten wurde der Autoklaveninhalt abgekühlt und das nicht umgesetzte Gasgemisch abgelüftet. Die so erhaltenen Reaktionsgemische wurden zur vollständigen Koagulation in 130 ml einer 4 %igen wäßrigen Magnesiumsulfatlösung gegossen. Die Produkte wurden mit Wasser gewaschen und dann getrocknet, wobei man Copolymere (weiße Pulver) bestehend aus Einheiten von Trifluorchlorethylen und 2-Allyl-octafluorcyclopentyltrifluorvinylether in den Ausbeuten und Zusammensetzungen (anhand der $^{19}$F-NMR Spektren oder Chlor-Elementaranalyse bestimmt) wie in Tabelle 1 angegeben erhielt.

Löslichkeiten, Grenzviskositätszahlen [$\eta$] und DSC-Ergebnisse sind ebenso in Tabelle 1 aufgeführt.

Ferner ist die Polymerzusammensetzung als Molverhältnis der aus Trifluorchlorethylen resultierenden Polymerbausteine zu den aus 2-Allyl-octafluorcyclopentyl-trifluorvinylether resultierenden Polymerbausteine angegeben.

**Tabelle 1**

| Bsp. | Monomer 1 | Polym.-zeit [h] | Ausbeute Polymer [g] | Polymerzusammensetz. [Molverh.] | [η] [dl/g] (Aceton 125° C | $T_g$ aus DSC [° C] | Löslichkeit[1)] |
|---|---|---|---|---|---|---|---|
| 7 | 7 | 10 | 16 | 90/10 | 0,065 | 60 | - |
| 8 | 10 | 10 | 12 | 79/21 | 0,063 | 64 | + |
| 9 | 20 | 20 | 27,5 | 67/33 | 0,052 | 72 | + |

1) - bedeutet, daß keine vollständige Lösung erhalten wird

\+ bedeutet, daß eine gelfreie Lösung erhalten wird

jeweils 25 mg Polymer/ml Aceton bei 25° C

## Patentansprüche

1. 2-Allyl-perfluoralkyltrifluorvinylether der Formel (I)

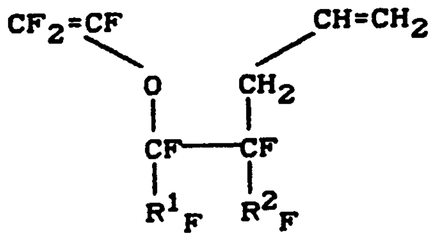

(I)

wobei

$R^1_F$ und $R^2_F$ entweder $-CF_3$ oder gemeinsam $(CF_2)_n$ mit n = 2 oder 3 bezeichnen.

2. 2-Allyl-perfluorcyclopentyltrifluorvinylether nach Anspruch 1.

3. Verfahren zur Herstellung von Verbindungen gemäß Anspruch 1, dadurch gekennzeichnet, daß

a) entsprechende 2-Allyl-substituierte Perfluorcycloketone bzw. Perfluorketone mit Hexafluorpropenoxid umgesetzt werden,

b) die erhaltenen 2-(Allylperfluorcycloalkoxy)- bzw. 2-)Allylperfluoralkoxy)-perfluorpropancarbonsäurefluoride durch alkalische Verseifung in deren Natriumsalze überführt werden und

c) die Natriumsalze bei 150 - 350 °C decarboxyliert werden.

4. Verfahren nach Anspruch 3, dadurch gekennzeichnet, daß zur Reduzierung der Entstehung von 2-Allylperfluoralkyl-1,1,1,2-Tetrafluorethylether als Nebenprodukt die unter b) erhaltenen Natriumsalze in aromatischen Kohlenwasserstoffren gelöst werden, unlösliche Salze aus der Lösung abgetrennt werden und die Natriumsalze azeotrop getrocknet werden.

**5.** Polymerisate bzw. Copolymerisate, enthaltend Polymorbausteine der Formeln

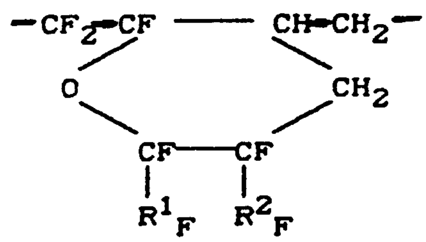

(II)

oder

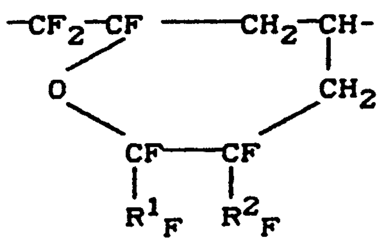

(III)

oder

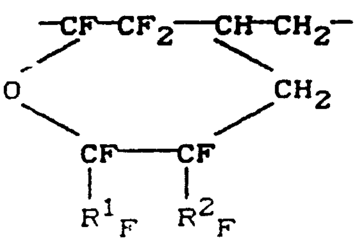

(IV)

wobei

$R^1_F$, $R^2_F$ entweder $-CF_3$ oder gemeinsam ( $CF_2$ ) n mit n = 2 oder 3 bezeichnen.

## Claims

**1.** 2-allyl-perfluoroalkyl-trifluorovinyl ethers of the formula (I)

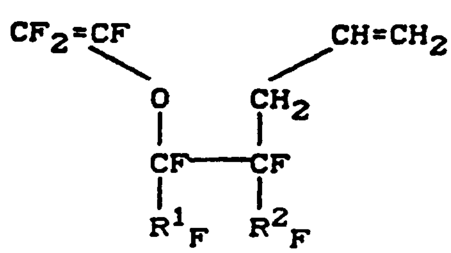

(I)

wherein

$R^1_F$ and $R^2_F$ represent either $-CF_3$ or, together, $(CF_2)_n$ where n = 2 or 3.

**2.** 2-allyl-perfluorocyclopentyl-trifluorovinyl ethers according to Claim 1.

**3.** A process for the preparation of compounds according to Claim 1, characterised in that

a) appropriate 2-allyl-substituted perfluorocycloketones or perfluoroketones are reacted with hexafluoropropene oxide,

b) the 2-(allylperfluorocycloalkoxy)- or 2-(allylperfluoro-alkoxy)-perfluoropropanoic acid fluorides are converted into their sodium salts by alkaline saponification and
c) the sodium salts are decarboxylated at 150 - 350 °C.

**4.** A process according to Claim 3, characterised in that, to reduce the production of 2-allyl-perfluoroalkyl-1,1,1,2-tetrafluoroethyl ethers as a side product, the sodium salts obtained under b) are dissolved in aromatic hydrocarbons, insoluble salts are separated from the solution and the sodium salts are dried azeotropically.

**5.** Polymers or copolymers, containing polymer structural units of the formulas

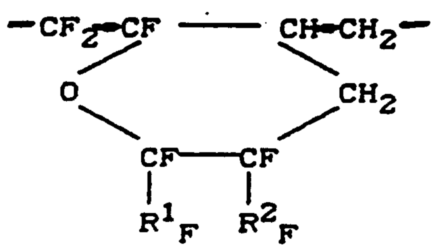

(II)

or

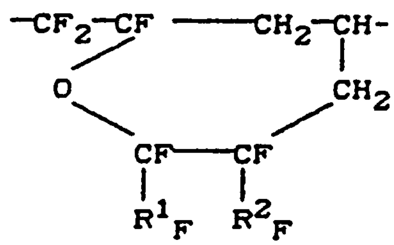

(III)

or

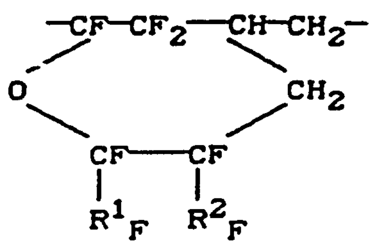

(IV)

wherein
$R^1_F$, $R^2_F$ represent either $-CF_3$ or, together, $(CF_2)_n$, where n = 2 or 3.

## Revendications

**1.** 2-allylperfluoralkyltrifluorovinyléthers répondant à la formule (I)

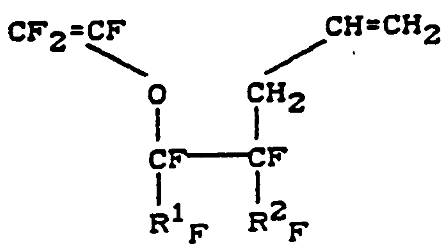

(I)

dans laquelle

$R^1_F$ et $R^2_F$, soit représentent $-CF_3$, soit représentent ensemble $(CF_2)_n$, n étant égal à 2 ou 3.

**2.** 2-allylperfluorocyclopentyltrifluorovinyléther selon la revendication 1.

**3.** Procédé pour la préparation de composés selon la revendication 1, caractérisé en ce que

a) on fait réagir des perfluorocyclocétones respectivement des perfluorocétones correspondantes substituées par un groupe 2-allyle avec de l'oxyde d'hexafluoropropène,

b) on transforme en leurs sels de sodium par saponification alcaline les fluorures d'acides 2-(allylperfluorocycloalcoxy)- respectivement 2-(allylperfluoralcoxy)-perfluoropropanecarboxyliques obtenus, et

c) on soumet les sels de sodium à une décarboxylation à une température de 150 - 350 °C.

**4.** Procédé selon la revendication 3, caractérisé en ce que, pour réduire l'apparition de 2-(allylperfluoralkyl)-1,1,1,2-tétrafluoréthyléthers comme sous-produit, on dissout les sels de sodium obtenus sous b) dans des hydrocarbures aromatiques, on sépare de la solution les sels insolubles et on sèche les sels de sodium par voie azéotrope.

**5.** Polymères respectivement copolymères contenant des constituants polymères répondant aux formules

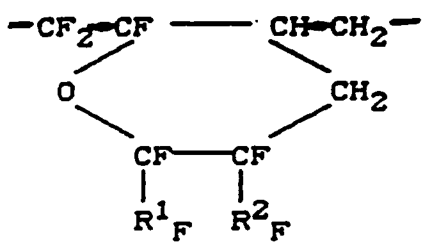

(II)

ou

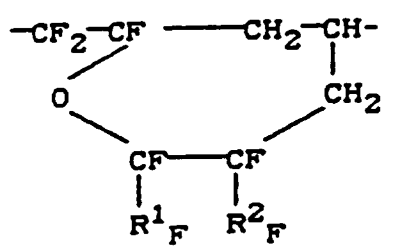

(III)

ou

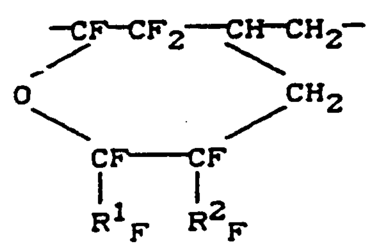

(IV)

dans lesquelles

$R^1_F$ et $R^2_F$, soit représentent $-CF_3$, soit représentent ensemble $(CF_2)_n$, n étant égal à 2 ou 3.